# FASCICULE DE BREVET EUROPEEN

(11) **EP 3 501 350 B1**
(45) Date de publication et mention de la délivrance du brevet: **19.08.2020**
(21) Numéro de dépôt: 18212915.5
(22) Date de dépôt: 17.12.2018
(51) Int. Cl.: A47J 31/52, G06F 21/32

(54) **PROCÉDÉ DE COMMANDE D'UNE MACHINE À CAFÉ EXPRESSO**
STEUERUNGSVERFAHREN EINER ESPRESSO-KAFFEEMASCHINE
METHOD FOR CONTROLLING AN ESPRESSO COFFEE MACHINE

(30) Priorité: 21.12.2017 FR 1762758
(43) Date de publication de la demande: 26.06.2019
(73) Titulaire: SEB S.A., 69130 Ecully (FR)
(72) Inventeur: GHECHOUA, Karim, 69800 SAINT PRIEST (FR)
(74) Mandataire: Soares, Luis Filipe

(56) Documents cités:
- EP-B1- 2 309 902
- WO-A1-2015/189148
- DE-A1-102014 205 809
- US-A1- 2012 285 986
- Foteini Agrafioti: "Rythme cardiaque", , 15 janvier 2018 (2018-01-15), XP055573107, Extrait de l'Internet: URL:https://web.archive.org/web/2018011511 2523/http://www.biometrie-online.net:80/te chnologies/rythme-cardiaque [extrait le 2019-03-22]

## Description

### Domaine de l'invention

La présente invention concerne un procédé de commande d'une machine à café expresso.

### Art antérieur

Il est connu de définir un profil utilisateur comprenant des préférences d'utilisation d'une machine à café expresso. Cette disposition permet à l'utilisateur dont le profil est défini d'avoir accès à des commandes spécifiques qu'il utilise régulièrement.

L'utilisateur doit alors se connecter sur son profil qui est enregistré sur la machine à café expresso. Ensuite, ses commandes favorites peuvent être mises à sa disposition par une interface utilisateur comme par exemple un écran tactile.

Ce mode de fonctionnement donne satisfaction en ce que l'utilisateur trouve facilement ses commandes préférées. Cependant l'utilisateur doit tout d'abord se connecter sur son profil ce qui nécessite des interactions avec l'interface utilisateur de sa part.

Ainsi le temps gagné par la mise à disposition des commandes préférées peut être contrebalancé par le temps nécessaire à la connexion au profil utilisateur.

Ceci est particulièrement vrai pour une machine à café expresso utilisée par plusieurs personnes car un maintien de la connexion sur un profil utilisateur donné entre deux utilisations n'est d'aucune utilité.

Il est également connu du document EP2309902B1 une machine à café expresso comprenant un système de reconnaissance pour reconnaitre au moins une caractéristique biométrique d'un utilisateur sur une machine à café expresso de manière à proposer directement un menu personnalisé à un utilisateur.

Une telle machine à café expresso est satisfaisante en ce que l'utilisateur n'a plus besoin de se connecter à un compte utilisateur pour que lui soit proposé un menu personnalisé.

Ce document cite toute une liste de systèmes de reconnaissance biométrique dont certains s'avèrent compliqués à mettre en œuvre sur une machine à café expresso, par exemple une identification ADN ou bien une lecture de l'iris d'un œil. D'autres systèmes de reconnaissance biométrique de cette liste apparaissent comme plus crédibles, comme l'identification d'empreintes digitales.

Le document DE102014205809A1 divulgue une machine à café avec un système d'identification de l'utilisateur.

La présente invention vise à proposer une alternative à ces derniers systèmes de reconnaissance biométrique.

### Exposé de l'invention

A cet effet, la présente invention concerne un procédé de commande d'une machine à café expresso comprenant au moins une première électrode et une seconde électrode, le procédé comprenant :
une étape de mise en contact de la première électrode avec un emplacement prédéterminé d'une partie droite du corps d'un utilisateur, et de la seconde électrode avec un emplacement prédéterminé d'une partie gauche du corps de l'utilisateur, la partie droite et la partie gauche du corps étant entendues comme étant respectivement du côté droit et du côté gauche du plan parallèle au plan sagittal du corps de l'utilisateur et traversant le cœur ;
une étape d'acquisition d'un signal électrocardiographique entre la première et la seconde électrode ;
une étape de sélection, à partir du signal électrocardiographique acquis, d'un identifiant unique particulier parmi une pluralité d'identifiants uniques enregistrés dans une base de données, chaque identifiant unique de la base de données étant associé d'une part à un signal électrocardiographique de référence enregistré dans la base de données et d'autre part à un profil utilisateur enregistré dans la base de données, ledit profil utilisateur comprenant des préférences d'utilisation de la machine à café expresso ;
une étape d'élaboration d'une information de commande, l'information de commande étant élaborée en fonction du profil utilisateur associé à l'identifiant unique sélectionné.

Cette disposition permet de simplifier la procédure d'élaboration de l'information de commande par l'utilisateur. En effet, le nombre d'interactions entre l'utilisateur et la machine à café expresso est limité puisque l'utilisateur n'a pas à entrer plusieurs commandes manuelles pour que l'information de commande soit élaborée.

Ainsi un utilisateur dont le profil est connu pourra commander de manière aisée la machine à café expresso puisque ses préférences d'utilisation sont connues.

Selon un aspect de l'invention, l'étape de sélection de l'identifiant unique particulier est établie selon une comparaison du signal électrocardiographique acquis avec les signaux électrocardiographiques de référence de la base de données.

Cette disposition permet de réaliser une identification simple, rapide et fiable dans le sens où la probabilité de confondre deux utilisateurs est limitée.

Selon un aspect de l'invention, l'information de commande correspond à une commande de mise à disposition par la machine à café expresso d'un élément d'interface utilisateur pourvu d'une sélection d'au moins une commande de fonctionnement parmi une pluralité de commandes de fonctionnement existantes de la machine à café expresso.

Cette disposition permet de limiter les choix proposés à l'utilisateur selon ses préférences d'utilisation. Aucune commande de fonctionnement que l'utilisateur ne sélectionne rarement ou qu'il ne souhaite pas utiliser n'est proposée.

Par exemple, seuls les types de cafés habituellement choisis par l'utilisateur sont proposés suite à son identification auprès de la machine.

Selon un aspect de l'invention, les étapes décrites ci-avant sont répétées, l'emplacement prédéterminé de la partie droite et respectivement l'emplacement prédéterminé de la partie gauche de l'utilisateur étant identiques à chaque étape d'acquisition d'un signal électrocardiographique.

Pour faire de l'identification par électrocardiographie, l'emplacement des mesures sur le corps doit être identique à chaque acquisition.

Les première et seconde électrodes utilisées pour de telles mesures sont passives. Une différence de potentiel est mesurée entre la partie droite et la partie gauche du corps de l'utilisateur par rapport au cœur. Le terme électrode est à comprendre comme plot conducteur.

Dans le corps humain, les muscles sont commandés, pilotés, par le système nerveux qui fait transiter des impulsions électriques. Le cœur étant un muscle, il travaille sous le rythme d'impulsions électriques. L'électrocardiographie consiste à capter ces impulsions électriques.

Le signal électrocardiographique dépend du positionnement des emplacements prédéterminés du corps sur lesquels sont mises en contact les première et seconde électrodes.

Afin de permettre une reconnaissance de l'utilisateur, les signaux électrocardiographiques doivent être obtenus dans les mêmes conditions que le signal de référence correspondant au profil utilisateur sélectionné.

Selon un aspect de l'invention, le procédé de commande comprend une étape de déclaration dans laquelle un profil utilisateur est créé et/ou modifié puis enregistré dans la base de données en association avec un identifiant unique, l'étape de déclaration étant réalisée préalablement à l'étape d'élaboration d'une information de commande.

Pour définir une information de commande en fonction de l'identifiant unique sélectionné, il est nécessaire au préalable d'associer un profil utilisateur à cet identifiant unique.

Selon un aspect de l'invention, le procédé de commande comprend une étape préalable d'initialisation dans laquelle un signal électrocardiographique de référence est mesuré entre la première et la seconde électrode respectivement positionnées sur l'emplacement prédéterminé de la partie droite et l'emplacement prédéterminé de la partie gauche du corps de l'utilisateur, ledit signal électrocardiographique de référence mesuré étant associé à un identifiant unique, cette association étant enregistrée dans la base de données.

Cette disposition permet de mémoriser la signature unique du signal électrocardiographique d'un utilisateur.

La présente invention concerne également un système électronique comprenant une machine à café expresso, le système électronique étant agencé pour mettre en œuvre les étapes d'un procédé de commande tel que décrit ci-avant et comprenant la première électrode, la seconde électrode et un étage de conditionnement pourvu d'un convertisseur analogique numérique apte à convertir le signal électrocardiographique acquis de type analogique en signal de type numérique.

Selon un aspect de l'invention, le système électronique comprend la base de données. Selon un autre aspect de l'invention, les signaux électrocardiographiques de référence sont de type numérique et de préférence sont enregistrés dans la base de données. Selon un aspect de l'invention, les profils utilisateur sont enregistrés dans la base de données.

Selon un aspect de l'invention, l'étage de conditionnement comprend un amplificateur d'instrumentation, en particulier à fort gain et/ou ayant un taux de réjection de mode commun élevé engendrant un signal différentiel à partir du signal électrocardiographique acquis.

Le signal différentiel est ensuite converti en signal numérique à l'aide du convertisseur analogique numérique, qui est de préférence de type différentiel.

Selon un aspect de l'invention, le système électronique comprend un étage décisionnel configuré pour la sélection, à partir du signal électrocardiographique acquis converti, d'un identifiant unique particulier parmi la pluralité d'identifiants uniques enregistrés dans la base de données, chaque identifiant unique de la base de données étant associé d'une part à un signal électrocardiographique de référence enregistré dans la base de données et d'autre part à un profil utilisateur enregistré dans la base de données, ledit profil utilisateur comprenant des préférences d'utilisation de la machine à café expresso.

Selon un aspect de l'invention, l'étage décisionnel est pourvu d'un calculateur comprenant une unité de traitement de l'information pour la comparaison du signal électrocardiographique acquis converti avec les signaux électrocardiographiques de référence.

De préférence l'unité de traitement de l'information est agencée pour appliquer un algorithme d'apprentissage automatique. En particulier, l'algorithme d'apprentissage automatique est enregistré en mémoire de l'unité de traitement de l'information. Ce type d'algorithme est également connu sous le terme anglais « machine learning ». Selon un aspect de l'invention, le calculateur comprend un processeur.

De préférence, l'application de l'algorithme d'apprentissage automatique correspond à une modélisation statistique de manière à reconnaître un signal électrocardiographique acquis parmi une pluralité de signaux électrocardiographiques acquis.

En particulier la modélisation statistique peut être appliquée par un arbre de décisions et/ou un réseau de neurones de l'unité de traitement de l'information.

Selon un aspect de l'invention, le système électronique comprend en outre un serveur distant, l'étage décisionnel étant compris dans le serveur distant.

Cette disposition permet de limiter la puissance de calcul de la machine à café expresso puisque les calculs sont déportés vers le serveur distant.

Selon un aspect de l'invention, la base de données est comprise dans le serveur distant. Cette disposition permet d'identifier un utilisateur avec toute machine à café expresso agencée pour échanger des informations avec le serveur distant.

Selon un aspect de l'invention, la première électrode et la seconde électrode sont ménagées dans des zones dédiées de la machine à café expresso.

Selon un aspect de l'invention, les zones dédiées correspondent à des boutons de commande de la machine à café expresso.

Selon un aspect de l'invention, les boutons de commande sont aptes à lancer une fonction de la machine à café expresso. Ainsi certaines tâches peuvent être automatisées comme le lancement de la préparation d'une boisson par la machine à café expresso.

Les différents aspects définis ci-dessus non incompatibles peuvent être combinés.

### Brève description des figures

L'invention sera mieux comprise à l'aide de la description détaillée qui est exposée ci-dessous en regard du dessin annexé dans lequel :
- la figure 1 est une vue schématique d'un système de commande,
- la figure 2 est un schéma des étapes d'un procédé de commande d'une machine à café expresso.

### Description en référence aux figures

Dans la description détaillée qui va suivre des figures définies ci-dessus, les mêmes éléments ou les éléments remplissant des fonctions identiques pourront conserver les mêmes références de manière à simplifier la compréhension de l'invention.

### Système électronique

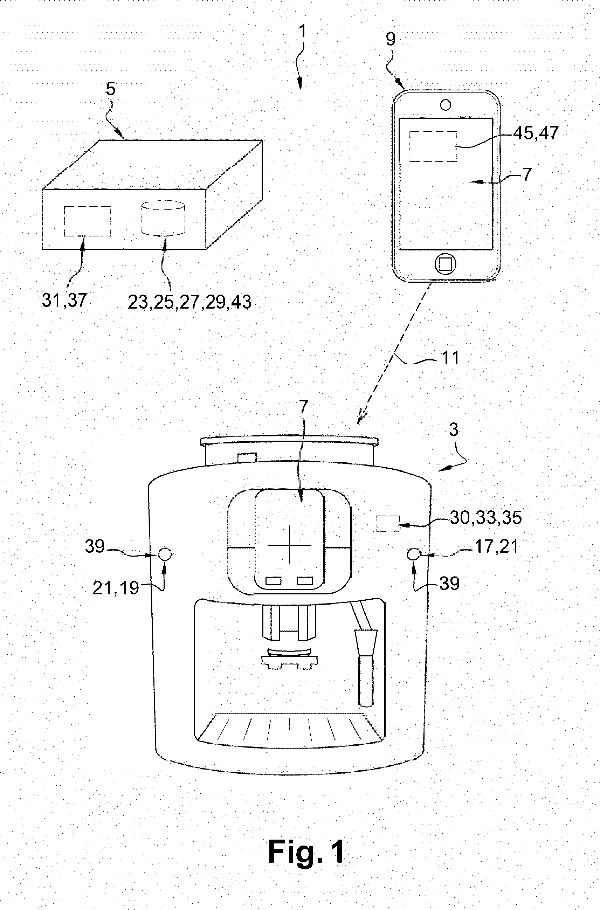

Comme illustré à la figure 1, un système électronique 1 comprend une machine à café expresso 3. Le système électronique 1 comprend également un serveur 5 distant et une interface utilisateur 7.

L'interface utilisateur 7 est ici partiellement déportée sur un terminal de communication 9 apte à échanger des informations à la fois avec le serveur 5 et la machine à café expresso 3. L'interface utilisateur 7 est également partiellement intégrée dans la machine à café expresso 3.

Ainsi la machine à café expresso 3 est agencée pour échanger des informations d'une part avec l'interface utilisateur 7 et d'autre part avec le serveur 5. La communication est réalisée sans fil par exemple selon un ou des protocoles de communication par ondes radio entre le terminal de communication 9, le serveur 5 et la machine à café expresso 3.

La machine à café expresso 3 est apte à réaliser une fonction suite à la réception d'une information de commande 11. A la figure 1, il s'agit de la distribution d'une boisson.

Le système électronique 1 comprend également une première électrode 17 et une deuxième électrode 19, lesdites électrodes 17, 19 étant configurées pour acquérir un signal électrocardiographique 21.

Le serveur 5 distant comprend une base de données 23 comprenant une pluralité d'identifiants uniques 25 enregistrés, chaque identifiant unique 25 étant associé à un signal électrocardiographique de référence 27 et un profil utilisateur 29 comprenant des préférences d'utilisation de la machine à café expresso 3.

Les signaux électrocardiographiques de références 27 et les profils utilisateurs 29 sont également enregistrés dans la base de données 23. Les signaux électrocardiographiques de références 27 sont sous forme de données numériques.

La machine à café expresso 3 comprend en outre un étage de conditionnement 30 pourvu d'un convertisseur analogique numérique 33 permettant de convertir le signal électrocardiographique acquis 21 par les électrodes 17, 19 en un signal numérique apte à être comparé avec un des signaux électrocardiographiques de référence 27.

L'étage de conditionnement 30 comprend un amplificateur d'instrumentation 35, en particulier à fort gain et/ou ayant un taux de réjection de mode commun élevé engendrant un signal différentiel à partir du signal électrocardiographique acquis 21.

Le signal différentiel est ensuite converti en signal numérique à l'aide du convertisseur analogique numérique 33, qui est de préférence de type différentiel.

Le serveur 5 comprend un étage décisionnel 31 pourvu d'un calculateur 37 comprenant une unité de traitement de l'information pour la comparaison du signal électrocardiographique acquis 21 converti avec les signaux électrocardiographiques de référence 27.

L'unité de traitement de l'information est également agencé pour comprendre en mémoire et appliquer un algorithme d'apprentissage automatique. Ce type d'algorithme est également connu sous le terme anglais « machine learning ».

### Procédé de commande

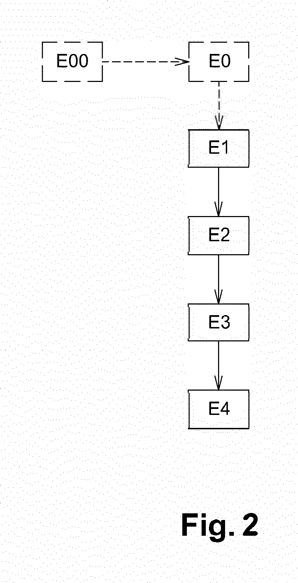

Comme illustré à la figure 2, un procédé de commande de la machine à café expresso 3 comprend les étapes détaillées ci-après.

Le procédé de commande comprend une étape E1 de mise en contact de la première électrode 17 avec un emplacement prédéterminé d'une partie droite du corps d'un utilisateur, et de la seconde électrode 19 avec un emplacement prédéterminé d'une partie gauche du corps de l'utilisateur.

La partie droite et la partie gauche du corps sont entendues comme étant respectivement du côté droit et du côté gauche du plan parallèle au plan sagittal du corps de l'utilisateur et traversant le cœur.

La première électrode 17 et la seconde électrode 19 sont ménagées dans des zones dédiées 39 de la machine à café expresso 3.

A la figure 1, les zones dédiées 39 correspondent à des boutons de commande de la machine à café expresso 3. Ces boutons sont distants de manière à forcer l'utilisateur à disposer son pouce gauche sur le bouton gauche et son pouce droit sur le bouton droit.

Ainsi les emplacements prédéterminés sont toujours les mêmes pour tous les utilisateurs.

Le procédé de commande comprend une étape E2 d'acquisition d'un signal électrocardiographique 21 entre la première électrode 17 et la seconde électrode 19.

Le procédé de commande comprend ensuite une étape E3 de sélection, à partir du signal électrocardiographique acquis 21, d'un identifiant unique 43 particulier parmi une pluralité d'identifiants uniques 43 enregistrés dans une base de données 23.

Chaque identifiant unique 43 de la base de données 23 est associé d'une part à un signal électrocardiographique de référence 27 enregistré dans la base de données 23 et d'autre part à un profil utilisateur 29 enregistré dans la base de données 23, ledit profil utilisateur 29 comprenant des préférences d'utilisation de la machine à café expresso 3.

La sélection de l'identifiant unique 43 particulier est établie selon une comparaison du signal électrocardiographique acquis 21 avec les signaux électrocardiographiques de référence 27 de la base de données 23.

L'utilisateur est ainsi reconnu par la signature unique de son signal électrocardiographique 21.

Le procédé comprend ensuite une étape E4 d'élaboration d'une information de commande 11, l'information de commande 11 étant élaborée en fonction du profil utilisateur 29 associé à l'identifiant unique 43 sélectionné.

A la figure 1, l'information de commande 11 dépendant des préférences utilisateur peut par exemple être une commande d'élaboration d'un expresso si l'utilisateur a défini ses préférences ainsi.

L'information de commande 11 peut également correspondre à une commande de mise à disposition par la machine à café expresso 3 d'un élément 45 d'interface utilisateur 7 pourvu d'une sélection d'au moins une commande de fonctionnement 47 parmi une pluralité de commandes de fonctionnement 47 existantes de la machine à café expresso 3.

Par exemple, seuls les types de cafés habituellement choisis par l'utilisateur sont proposés suite à son identification auprès de la machine à café.

Les étapes E1 à E4 peuvent être répétées, l'emplacement prédéterminé de la partie droite et respectivement l'emplacement prédéterminé de la partie gauche de l'utilisateur étant identiques à chaque étape d'acquisition d'un signal électrocardiographique 21.

Cette disposition est particulièrement intéressante lorsque plusieurs utilisateurs se servent à tour de rôle de la machine à café expresso 3.

Dans le cas de la figure 1, chaque utilisateur peut ainsi avoir rapidement son café selon ses préférences et laisser la place libre pour les utilisateurs suivants.

Il est nécessaire de créer préalablement un profil utilisateur 29 et également de définir les préférences utilisateurs liées, ces préférences pouvant être modifiées ultérieurement.

Pour ce faire le procédé de commande comprend une étape E0 de déclaration dans laquelle un profil utilisateur 29 est créé et/ou modifié puis enregistré dans la base de données 23 en association avec un identifiant unique 43.

Le procédé comprend en outre une étape E00 préalable d'initialisation dans laquelle un signal électrocardiographique de référence 27 est mesuré entre la première électrode 17 et la seconde électrode 19 respectivement positionnées sur l'emplacement prédéterminé de la partie droite et l'emplacement prédéterminé de la partie gauche du corps de l'utilisateur, ledit signal électrocardiographique de référence 27 mesuré étant associé à un identifiant unique 43, cette association étant enregistrée dans la base de données 23.

Cette manipulation peut être réalisée par interaction avec l'interface utilisateur 7, l'affichage de l'interface utilisateur 7 pouvant guider l'utilisateur dans la configuration de son profil 29.

Ainsi une fois le profil utilisateur 29 défini, l'identification de l'utilisateur auprès de la machine à café expresso 3 est très rapide. Il n'y a aussi aucun risque d'être connecté avec un mauvais profil utilisateur 29, par exemple sous le profil 29 de l'utilisateur précédent.

Comme il va de soi, l'invention ne se limite pas à la seule forme d'exécution décrite ci-dessus à titre d'exemple, elle en embrasse au contraire toutes les variantes de réalisation.

## Revendications

1. Procédé de commande d'une machine à café expresso (3) comprenant au moins une première électrode (17) et une seconde électrode (19), le procédé comprenant :
- (E1) une étape de mise en contact de la première électrode (17) avec un emplacement prédéterminé d'une partie droite du corps d'un utilisateur, et de la seconde électrode avec un emplacement prédéterminé d'une partie gauche du corps de l'utilisateur, la partie droite et la partie gauche du corps étant entendues comme étant respectivement du côté droit et du côté gauche du plan parallèle au plan sagittal du corps de l'utilisateur et traversant le cœur ;
- (E2) une étape d'acquisition d'un signal électrocardiographique (21) entre la première et la seconde électrode (17, 19) ;
- (E3) une étape de sélection, à partir du signal électrocardiographique acquis (21), d'un identifiant unique (43) particulier parmi une pluralité d'identifiants uniques (43) enregistrés dans une base de données (23), chaque identifiant unique (43) de la base de données (23) étant associé d'une part à un signal électrocardiographique de référence (27) enregistré dans la base de données (23) et d'autre part à un profil utilisateur (29) enregistré dans la base de données (23), ledit profil utilisateur (29) comprenant des préférences d'utilisation de la machine à café expresso (3) ;
- (E4) une étape d'élaboration d'une information de commande (11), l'information de commande (11) étant élaborée en fonction du profil utilisateur (29) associé à l'identifiant unique (43) sélectionné.

2. Procédé de commande selon la revendication 1, dans lequel l'étape (E3) de sélection de l'identifiant unique (43) particulier est établie selon une comparaison du signal électrocardiographique acquis (21) avec les signaux électrocardiographiques de référence (27) de la base de données (23).

3. Procédé de commande selon l'une des revendications 1 ou 2, dans lequel l'information de commande (11) correspond à une commande de mise à disposition par la machine à café expresso (3) d'un élément (45) d'interface utilisateur (7) pourvu d'une sélection d'au moins une commande de fonctionnement (47) parmi une pluralité de commandes de fonctionnement (47) existantes de la machine à café expresso (3).

4. Procédé de commande selon l'une des revendications 1 à 3, dans lequel les étapes de la revendication 1 sont répétées, l'emplacement prédéterminé de la partie droite et respectivement l'emplacement prédéterminé de la partie gauche de l'utilisateur étant identiques à chaque étape (E2) d'acquisition d'un signal électrocardiographique (21).

5. Procédé de commande selon l'une des revendications 1 à 4, comprenant une étape de déclaration (E0) dans laquelle un profil utilisateur (29) est créé et/ou modifié puis enregistré dans la base de données (23) en association avec un identifiant unique (43), l'étape de déclaration (E0) étant réalisée préalablement à l'étape (E4) d'élaboration d'une information de commande (11).

6. Procédé de commande selon l'une des revendications 1 à 5, comprenant une étape préalable d'initialisation (E00) dans laquelle un signal électrocardiographique de référence (27) est mesuré entre la première et la seconde électrode (17, 19) respectivement positionnées sur l'emplacement prédéterminé de la partie droite et l'emplacement prédéterminé de la partie gauche du corps de l'utilisateur, ledit signal électrocardiographique de référence (27) mesuré étant associé à un identifiant unique (43), cette association étant enregistrée dans la base de données (23).

7. Système électronique (1) comprenant une machine à café expresso (3), le système électronique (1) étant agencé pour mettre en œuvre les étapes d'un procédé selon l'une des revendications 1 à 6 et comprenant la première électrode (17), la seconde électrode (19) et un étage de conditionnement (30) pourvu d'un convertisseur analogique numérique (33) apte à convertir le signal électrocardiographique (21) acquis de type analogique en signal de type numérique.

8. Système électronique (1) selon la revendication 7, comprenant un étage décisionnel (31) configuré pour la sélection, à partir du signal électrocardiographique acquis (21) converti, d'un identifiant unique (43) particulier parmi la pluralité d'identifiants uniques (43) enregistrés dans la base de données (23), chaque identifiant unique (43) de la base de données (23) étant associé d'une part à un signal électrocardiographique de référence (27) enregistré dans la base de données (23) et d'autre part à un profil utilisateur (29) enregistré dans la base de données (23), ledit profil utilisateur (29) comprenant des préférences d'utilisation de la machine à café expresso (3).

9. Système électronique (1) selon la revendication 8, dans lequel l'étage décisionnel (31) est pourvu d'un calculateur comprenant une unité de traitement de l'information pour la comparaison du signal électrocardiographique acquis (21) converti avec les signaux électrocardiographiques de référence (27).

10. Système électronique (1) selon l'une des revendications 8 à 9, dans lequel la première électrode (17) et la seconde électrode (19) sont ménagées dans des zones dédiées (39) de la machine à café expresso (3).

11. Système électronique (1) selon la revendication 10, dans lequel les zones dédiées (29) correspondent à des boutons de commande de la machine à café expresso (3).

## Patentansprüche

1. Steuerverfahren einer Espressomaschine (3), umfassend mindestens eine erste Elektrode (17) und eine zweite Elektrode (19), wobei das Verfahren umfasst:
- (E1) einen Schritt zum Kontaktieren der ersten Elektrode (17) mit einer vorbestimmten Stelle eines rechten Körperteils eines Benutzers und der zweiten Elektrode mit einer vorbestimmten Stelle eines linken Körperteils des Benutzers, wobei das rechte Körperteil und das linke Körperteil als auf der rechten Seite bzw. auf der linken Seite der Ebene parallel zu der Sagittalebene des Körpers des Benutzers und das Herz kreuzend verstanden werden;
- (E2) einen Schritt zum Erfassen eines elektrokardiographischen Signals (21) zwischen der ersten und der zweiten Elektrode (17, 19);
- (E3) einen Schritt zum Auswählen einer bestimmten, eindeutigen Kennung (43) aus mehreren, eindeutigen Kennungen (43), die in einer Datenbank (23) gespeichert sind, ausgehend von dem erfassten elektrokardiographischen Signal (21), wobei jede eindeutige Kennung (43) der Datenbank (23) einerseits einem elektrokardiographischen Referenzsignal (27), das in der Datenbank (23) gespeichert ist, und andererseits einem Benutzerprofil (29), das in der Datenbank (23) gespeichert ist, zugeordnet ist, wobei das Benutzerprofil (29) Präferenzen für die Verwendung der Espressomaschine (3) umfasst;
- (E4) einen Schritt zum Entwickeln von einer Steuerinformation (11), wobei die Steuerinformation (11) als Funktion des Benutzerprofils (29) entwickelt ist, das der ausgewählten eindeutigen Kennung (43) zugeordnet ist.

2. Steuerverfahren nach Anspruch 1, wobei der Schritt (E3) zum Auswählen der bestimmten eindeutigen Kennung (43) gemäß einem Vergleich des erfassten elektrokardiographischen Signals (21) mit den elektrokardiographischen Referenzsignalen (27) der Datenbank (23) ermittelt wird.

3. Steuerverfahren nach einem der Ansprüche 1 und 2, wobei die Steuerinformation (11) einem Befehl zum Bereitstellen eines Elements (45) einer Benutzerschnittstelle (7), das für eine Auswahl von mindestens einem Betriebsbefehl (47) vorgesehen ist, durch die Espressomaschine (3) aus mehreren vorhandenen Betriebsbefehlen (47) der Espressomaschine (3) entspricht.

4. Steuerverfahren nach einem der Ansprüche 1 bis 3, wobei die Schritte von Anspruch 1 wiederholt werden, wobei die vorbestimmte Stelle des rechten Teils bzw. die vorbestimmte Stelle des linken Teils des Benutzers identisch mit jedem Schritt (E2) zum Erfassen eines elektrokardiographischen Signals (21) sind.

5. Steuerverfahren nach einem der Ansprüche 1 bis 4, umfassend einen Deklarationsschritt (E0), in dem ein Benutzerprofil (29) erstellt und / oder geändert und dann in Verbindung mit einer einmaligen Kennung (43) in der Datenbank (23) gespeichert wird, wobei der Deklarationsschritt (E0) vor dem Schritt (E4) Schritt zum Entwickeln von einer Steuerinformation (11) ausgeführt wird.

6. Steuerverfahren nach einem der Ansprüche 1 bis 5, umfassend einen vorläufigen Initialisierungsschritt (E00), wobei ein elektrokardiographisches Referenzsignal (27) zwischen der ersten und der zweiten Elektrode (17, 19) gemessen wird, die jeweils auf der vorbestimmten Stelle des rechten Teils und der vorbestimmten Stelle des linken Teils des Körpers des Benutzers positioniert sind, wobei das gemessene elektrokardiographische Referenzsignal (27) einer eindeutigen Kennung (43) zugeordnet ist, wobei diese Zuordnung in der Datenbank (23) gespeichert ist.

7. Elektronisches System (1), umfassend eine Espressomaschine (3), wobei das elektronische System (1) so angeordnet ist, dass es die Schritte eines Verfahrens nach einem der Ansprüche 1 bis 6 ausführt, und die erste Elektrode (17), die zweite Elektrode (19) und eine Konditionierungsstufe (30) umfasst, die mit einem analogen digitalen Wandler (33) versehen ist, der das erfasste elektrokardiographische Signal (21) vom analogen Typ in ein Signal vom digitalen Typ umwandeln kann.

8. Elektronisches System (1) nach Anspruch 7, umfassend eine Entscheidungsstufe (31), die ausgehend von dem umgewandelten elektrokardiographischen Signal (21) für die Auswahl einer bestimmten eindeutigen Kennung (43) aus der Vielzahl eindeutiger Kennungen (43), die in der Datenbank (23) gespeichert sind, konfiguriert ist, wobei jede eindeutige Kennung (43) der Datenbank (23) einerseits einem elektrokardiographischen Referenzsignal (27), das in der Datenbank (23) gespeichert ist, und andererseits einem Benutzerprofil (29) zugeordnet ist, das in der Datenbank (23) gespeichert ist, wobei das Benutzerprofil (29) Präferenzen für die Verwendung der Espressomaschine (3) umfasst.

9. Elektronisches System (1) nach Anspruch 8, wobei die Entscheidungsstufe (31) mit einem Rechner versehen ist, der eine Informationsverarbeitungseinheit zum Vergleich des umgewandelten, erfassten, elektrokardiographischen Signals (21) mit den elektrokardiographischen Referenzsignalen (27) umfasst.

10. Elektronisches System (1) nach einem der Ansprüche 8 bis 9, wobei die erste Elektrode (17) und die zweite Elektrode (19) in zweckgebundenen Bereichen (39) der Espressomaschine (3) untergebracht sind.

11. Elektronisches System (1) nach Anspruch 10, wobei die zweckgebundenen Bereiche (29) Steuertasten der Espressomaschine (3) entsprechen.

## Claims

1. Method for controlling an espresso coffee machine (3) comprising at least one first electrode (17) and a second electrode (19), the method comprising:
- (E1) a step of putting into contact the first electrode (17) with a predetermined placement of a right-hand portion of the body of a user, and of the second electrode with a predetermined placement of a left-hand portion of the body of the user, the right-hand portion and the left-hand portion of the body being understood as being respectively on the right-hand side and on the left-hand side of the plane parallel to the sagittal plane of the body of the user and passing through the core;
- (E2) a step of acquiring an electrocardiographic signal (21) between the first and the second electrode (17, 19);
- (E3) a step of selecting, from the electrocardiographic signal acquired (21), from a unique particular identifier (43) from among a plurality of unique identifiers (43) recorded in a database (23), each unique identifier (43) of the database (23) being associated, on the one hand, with a reference electrocardiographic signal (27) recorded in the database (23), and on the other hand, with a user profile (29) recorded in the database (23), said user profile (29) comprising preferences of using the espresso coffee machine (3);
- (E4) a step of developing an item of control information (11), the control information (11) being developed according to the user profile (29) associated with the unique identifier (43) selected.

2. Control method according to claim 1, wherein step (E3) for selecting the particular unique identifier (43) is established according to a comparison of the electrocardiographic signal acquired (21) with the reference electrocardiographic signals (27) of the database (23).

3. Control method according to one of claims 1 or 2, wherein the control information (11) corresponds to a control for provisioning, by the espresso coffee machine (3), a user interface (7) element (45) provided with a selection of at least one operating command (47) from among a plurality of current operating commands (47) of the espresso coffee machine (3).

4. Control method according to one of claims 1 to 3, wherein the steps of claim 1 are repeated, the predetermined placement of the right-hand portion and respectively the predetermined placement of the left-hand portion of the user being identical to each step (E2) of acquiring an electrocardiographic signal (21).

5. Control method according to one of claims 1 to 4, comprising a declaration step (E0) wherein a user profile (29) is created and/or modified, then recorded in the database (23) in association with a unique identifier (43), the declaration step (E0) being carried out prior to step (E4) of developing an item of control information (11).

6. Control method according to one of claims 1 to 5, comprising a prior initialisation step (E00), wherein a reference electrocardiographic signal (27) is measured between the first and the second electrode (17, 19) respectively positioned on the predetermined placement of the right-hand portion and the predetermined placement of the left-hand portion of the body of the user, said reference electrocardiographic signal (27) measured being associated with a unique identifier (43), this association being recorded in the database (23).

7. Electronic system (1) comprising an espresso coffee machine (3), the electronic system (1) being arranged to implement the steps of a method according to one of claims 1 to 6 and comprising the first electrode (17), the second electrode (19) and a conditioning stage (30) provided with an analogue-to-digital converter (33) capable of converting the electrocardiographic signal (21) acquired of analogue type into a digital-type signal.

8. Electronic system (1) according to claim 7, comprising a decision-making stage (31) configured to select, from the converted, acquired electrocardiographic signal (21), a particular unique identifier (43) from among the plurality of unique identifiers (43) recorded in the database (23), each unique identifier (43) of the database (23) being associated, on the one hand, with a reference electrocardiographic signal (27) recorded in the database (23), and on the other hand, with a user profile (29) recorded in the database (23), said user profile (29) comprising preferences for using the espresso coffee machine (3).

9. Electronic system (1) according to claim 8, wherein the decision-making stage (31) is provided with a calculator comprising a unit for processing the information for comparing the converted, acquired electrocardiographic signal (21) with the reference electrocardiographic signals (27).

10. Electronic system (1) according to one of claims 8 to 9, wherein the first electrode (17) and the second electrode (19) are arranged in dedicated zones (39) of the espresso coffee machine (3).

11. Electronic system (1) according to claim 10, wherein the dedicated zones (29) correspond to buttons for controlling the espresso coffee machine (3).
